# EUROPEAN PATENT APPLICATION

(11) **EP 1 354 584 A1**
(43) Date of publication of application: **22.10.2003**
(21) Application number: 01995025.2
(22) Date of filing: 27.12.2001
(51) Int. Cl.: A61K 31/401, A61P 19/02, A61P 29/00, A23L 1/305, A23K 1/16, C07D 207/16

(54) **PREVENTIVES FOR ARTHRITIS**

(30) Priority: 05.01.2001 JP 2001000393
(71) Applicant: Kyowa Hakko Kogyo Co., Ltd, Tokyo 100-8185 (JP)
(72) Inventor: NAKAGIRI, Ryusuke, c/o Tsukuba Research Lab., Tsukuba-shi, Ibaraki 305-0841 (JP); KAMIYA, Toshikazu, c/o Tsukuba Research Lab., Tsukuba-shi, Ibaraki 305-0841 (JP)
(74) Representative: VOSSIUS & PARTNER
(86) International application number: JP0111540
(87) International publication number: WO02055073

(57) **Abstract**

The present invention relates to a preventive against arthritis, foods and drinks, food additives, animal feeds and feed additives comprising an N-acylated hydrxyproline derivative or a salt thereof as an active ingredient, use of an N-acylated hydroxyproline derivative or a salt thereof for the production of a preventive against arthritis and a method for preventing arthritis which comprises administering an N-acylated hydroxyproline derivative or a salt thereof.

## Description

### Technical Field

The present invention relates to a preventive against arthritis, foods and drinks, food additives, animal feeds, feed additives and a method for preventing arthritis by' using them.

### Background Art

With changes in our lifestyle and aging of the population, arthritis is expected to increase in the future.

N-Acetylhydroxyproline is known as a chemical substance that shows anti-inflammatory activity (US Patent Nos. 3,891,765 and 3,932,638; Japanese Published Examined Patent Application No. 43947/72).

It is known that administration of N-acetylhydroxyproline to an animal model for arthritis after induction of arthritis can prevent aggravation of arthritis [J. Drug. Dev., 3, 135-142 (1990)]. It is also known that although N-acetylhydroxyproline shows a therapeutic effect on systemic injury of ear and tail, it shows no effect on chondral injury of at joints [Pharmacological Research, 33, 367-373 (1996)].

However, it has not been known so far that N-acylated hydroxyproline derivatives such as N-acetylhydroxyproline show a preventive effect on arthritis.

### Disclosure of the Invention

An object of the present invention is to provide a preventive against arthritis, a food and drink, a food additive, an animal feed and a feed additive comprising an N-acylated hydroxyproline derivative or a salt thereof as an active ingredient and a method for preventing arthritis by using them.

The present invention relates to the following (1)-(15).
(1) A preventive against arthritis comprising an N-acylated hydroxyproline derivative or a salt thereof as an active ingredient.
(2) The preventive against arthritis according to the above (1), wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.
(3). The preventive against arthritis according to the above (1) or (2), wherein the acyl moiety of said N-acylated hydroxyproline derivative is an acyl group having 2-23 carbon atoms.
(4) The preventive against arthritis according to any one of the above (1) to (3), wherein said N-acylated hydroxyproline derivative is N-acetylhydroxyproline.
(5) The preventive against arthritis according to any one of the above (1) to (4), wherein said arthritis is rheumatoid arthritis.
(6) A food and drink or an animal feed comprising an N-acylated hydroxyproline derivative or a salt thereof.
(7) The food and drink or the animal feed according to the above (6), wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.
(8) The food and drink or the animal feed according to the above (6) or (7), wherein the acyl moiety of said N-acylated hydroxyproline derivative is an acyl group having 2-23 carbon atoms.
(9) The food and drink or the animal feed according to any one of the above (6) to (8), wherein said N-acylated hydroxyproline derivative is N-acetylhydroxyproline.
(10) A food additive or a feed additive comprising an N-acylated hydroxyproline derivative or a salt thereof.
(11) The food additive or the feed additive according to the above (10), wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.
(12) The food additive or the feed additive according to the above (10) or (11), wherein the acyl moiety of said N-acylated hydroxyproline derivative is an acyl group having 2-23 carbon atoms.
(13) The food additive or the feed additive according to any one of the above (10) to (12), wherein said N-acylated hydroxyproline derivative is N-acetylhydroxyproline.
(14) Use of an N-acylated hydroxyproline derivative or a salt thereof for the production of a preventive against arthritis.
(15) A method for preventing arthritis, which comprises administering an N-acylated hydroxyproline derivative or a salt thereof.

Hydroxyproline widely occurs in nature as a major amino acid component of collagen and as an amino acid component of elastin. It is known that there exist eight kinds of stereoisomers of natural hydroxyproline, which are distinct in the following points: proline is the D-form or the L-form, the hydroxyl group is at the 3-position or the 4-position, and the stereoisomer is the cis-form or the trans-form, examples thereof being cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline. Although hydroxyproline of any such structure can be used in the present invention, trans-4-hydroxy-L-proline is preferably used.

Hydroxyproline can be obtained by subjecting collagen derived from animals such as pig and cow to acid hydrolysis and purifying the hydrolysate according to a conventional method. However, hydroxyproline produced using microorganisms is more preferably used.

Useful microorganisms include those belonging to the genus selected from the group consisting of Amycolatopsis, Dactylosporangium and Streptomyces or those into which a proline 3-hydroxylase gene or a proline 4-hydroxylase gene derived from these microorganisms has been introduced.

Introduction of a proline 3-hydroxylase gene or a proline 4-hydroxylase gene derived from a microorganism belonging to the genus selected from the group consisting of Amycolatopsis, Dactylosporangiurn and Streptomyces into a microorganism can be carried out according to the methods described in Molecular Cloning, A Laboratory Manual, Second Edition, Cold spring Harbor Laboratory Press (1989), Current Protocols in Molecular Biology, John Wiley & Sons (1987-1997), etc.

Furthermore, trans-4-hydroxy-L-proline can be produced using proline 4-hydroxylase isolated from a microorganism belonging to the genus Amycolatopsis or Dactylosporangium (Japanese Published Unexamined Patent Application No. 313179/95), and cis-3-hydroxy-L-proline can be produced using proline 3-hydroxylase isolated from a microorganism belonging to the genus Streptomyces (Japanese Published Unexamined Patent Application No. 322885/95) [Bioindustry, 14, 31 (1997)].

The acyl moiety of the N-acylated hydroxyproline derivatives used in the present invention includes straight-chain or branched acyl groups having 2-23 carbon atoms, for example, acetyl, propionyl, butyryl, isobutyryl, valeryl, hexanoyl, heptanoyl, octanoyl, decanoyl and eicosanoyl, among which acetyl and propionyl are preferred.

The N-acylated hydroxyproline derivatives can be produced according to a known method.

That is, the N-acylated hydroxyproline derivatives can be prepared by N-acylating hydroxyproline in an aqueous medium or an organic solvent using an active derivative (acid anhydride, acid chloride, etc.) of a fatty acid having an alkyl group having preferably 1-22 carbon atoms.

The N-acylated hydroxyproline derivatives thus obtained can be purified by conventional purification methods such as crystallization and chromatography.

Examples of the salts of N-acylated hydroxyproline derivatives include alkali metal salts such as sodium salts and potassium salts, alkaline earth metal salts such as magnesium salts and calcium salts; ammonium salts such as ammonium salts and tetramethylammonium salts, and organic amine addition salts such as salts with morpholine and piperidine.

The present invention can be applied to any arthritis such as chlamydial arthritis, chronic absorptive arthritis, enteropathic arthritis, gonococcal arthritis, gouty arthritis, Jaccoud arthritis, juvenile arthritis, Lyme arthritis, ochronotic arthritis, suppurative arthritis, osteoarthritis, periarthritis scapulohumeralis (so-called frozen shoulder), arthritis caused by excessive work load and rheumatoid arthritis, and is particularly advantageous to rheumatoid arthritis.

The preventive against arthritis, the foods and drinks, the animal feeds, the food additives and the feed additives of the present invention and the method for preventing arthritis in humans or non-human animals using them are described below.
(a) Preventive against arthritis comprising an N-acylated hydroxyproline derivative or a salt thereof as an active ingredient and method for preventing arthritis in humans or non-human animals using the preventive

The preventive against arthritis of the present invention comprises an N-acylated hydroxyproline derivative or a salt thereof as an active ingredient.

The preventive against arthritis of the present invention also includes mixtures of an N-acylated hydroxyproline derivative or a salt thereof with other optional ingredients that are effective in preventing arthritis (hereinafter also referred to simply as "Other active ingredients").

Other active ingredients include purified products or extracts of, or products containing boron, calcium, chromium, copper, magnesium, manganese, selenium, silicone, zinc, S-adenosyl methionine, collagen, collagen hydrolysate, gelatin, gelatin hydrolysate, bromelain, trypsin, chymotrypsin, papain, rutin, carotenoid, flavonoid, antioxidant vitamins, γ-linolenic acid, eicosapentaenoic acid, boswellia, capsaicin, cat's claw, devil's claw, fever few, ginger, nettles, niacinamide, shark cartilage, turmeric, curcumin, amino sugar, glycosaminoglycan, and the like.

The preventive against arthritis of the present invention is produced by optional methods well known in the technical field of pharmaceutics by mixing an N-acylated hydroxyproline derivative or a salt thereof and, if necessary, the above-described other active ingredients with one or more pharmaceutically acceptable carriers.

In administering the preventive against arthritis of the present invention, it is desirable to select a route of administration that is effective in the prevention of arthritis, examples thereof being oral administration and parenteral administrations such as intravenous administration.

Examples of the dosage form include tablets, capsules, injections, drops, syrups, sublingual tablets, various types of creams and suppositories.

Liquid preparations such as syrups that are suitable for oral administration can be produced using water, sugars such as sucrose, sorbitol and fructose, glycols such as polyethylene glycol and propylene glycol, oils such as sesame oil, olive oil and soybean oil, antiseptics such as p-hydroxybenzoic acid esters, flavors such as strawberry flavor and peppermint, etc. Furthermore, tablets, powders and granules can be produced using excipients such as lactose, glucose, sucrose and mannitol, disintegrators such as starch and sodium alginate, lubricants such as magnesium stearate and talc, binders such as polyvinyl alcohol, hydroxypropyl cellulose and gelatin, surfactants such as fatty acid ester, plasticizers such as glycerin, etc.

Preparations appropriate for parenteral administration comprise, preferably, a sterilized aqueous agent containing an active compound, which is isotonic to the recipient's blood. In the case of an injection, for example, an injectable solution is prepared using a carrier consisting of a salt solution, a glucose solution or a mixture of saline and a glucose solution.

In producing these parenteral preparations, it is also possible to add one or more supplementary components selected from diluents, antiseptics, flavors, excipients, disintegrators, lubricants, binders, surfactants, plasticizers and the like exemplified in connection with oral preparations.

The content of an N-acylated hydroxyploline derivative or a salt thereof in the preventive against arthritis of the present invention is 1-1000 mg/g, preferably 10-500 mg/g, especially preferably 100-200 mg/g of the preventive against arthritis.

The dosage and the frequency of administration of the preventive against arthritis of the present invention vary depending on the mode of administration, the age, the body weight and the symptoms of the patient.

In the case of oral administration, the preventive is administered in a dose of 1-5000 mg, preferably 10-1000 mg, especially preferably 100-500 mg as an N-acylated hydroxyproline derivative or a salt thereof per adult person once to several times a day.

In the case of parenteral administration such as intravenous administration, the preventive is administered in a dose of 0.5-5000 mg, preferably 5-1000 mg, especially preferably 50-500 mg as an N-acylated hydroxyproline derivative or a salt thereof per adult person once to several times a day.

By administering the preventive against arthritis of the present invention on a daily basis, it is possible to prevent arthritis.

The term "to prevent arthritis" as used herein means bringing about an effect of completely preventing the development of arthritis, reducing the incidence or suppressing the symptoms at the time of onset by taking the foods and drinks, animal feeds, food additives or feed additives to be explained in (b) below or the above-described preventive on a daily basis.

That is, prevention of arthritis is clearly distinguished from treatment of arthritis which relieves or treats symptoms by administering a drug after the onset of arthritis.

The preventive against arthritis of the present invention can be used not only for humans but also for animals other than humans (non-human animals), when used for non-human animals, the dosage is 0.02-100 mg/kg, preferably 0.2-20 mg/kg, especially preferably 2-10 mg/kg as an N-acylated hydroxyproline derivative or a salt thereof.
(b) Foods and drinks, animal feeds, food additives and feed additives comprising an N-acylated hydroxyproline derivative or a salt thereof and method for preventing arthritis in humans or non-human animals using them

The foods and drinks of the present invention are obtained by adding an N-acylated hydroxyproline derivative or a salt thereof to foods and drinks.

The foods and drinks of the present invention also include those obtainable by the addition of the food additives of the present invention.

The foods and drinks of the present invention further include those obtainable by adding other active ingredients described in (a) above in addition to an N-acylated hydroxyproline derivative or a salt thereof.

Except the addition of an N-acylated hydroxyproline derivative or a salt thereof, the foods and drinks of the present invention can be prepared using a conventional process for producing a food and drink.

The foods and drinks of the present invention may be in any of the forms including juice, soft drinks, tea, lactic acid beverages, fermented milk, ices, dairy products such as butter, cheese, yogurt, processed milk and skim milk, meat products such as ham, sausages and hamburger, fish products such as steamed, baked or fried fish paste, egg products such as baked or steamed foods made of beaten eggs, confectionery such as cookies, jellies, chewing gum, candies and snacks, bread, noodles, pickles, smoked foods, dried fish, preserved foods boiled down in soy sauce, salted foods, soups and seasonings.

Furthermore, the foods and drinks of the invention may take the form of a powdered food, a sheet-shaped food, a bottled food, a canned food, a retort food, a capsule food, a tablet food, a liquid food, a health drink, etc.

The foods and drinks of the present invention can be used as a health food or a functional food having an effect on the prevention of arthritis.

When the foods and drinks of the present invention are a drink or a tablet, they can be prepared by adding, to an N-acylated hydroxyproline derivative or a salt thereof, other active ingredients, additives, etc. as required and then dissolving or dispersing the mixture in an appropriate amount of water or tableting the mixture. Furthermore, when the foods and drinks of the present invention are confectionary such as caramels, drops, chocolate, jelly, biscuits and cookies, they can be prepared by adding, to an N-acylated hydroxyproline derivative or a salt thereof, other active ingredients, additives, etc. as required and additionally appropriate carriers such as wheat flour, rice flour, starch, corn starch, soybean, etc. as required and shaping the obtained mixture into an appropriate form according to a conventional method.

The foods and drinks of the present invention can also be produced by using granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method, and extrusion methods using an extruding granulator or an extruder.

The food additives of the present invention can be prepared according to methods similar to those mentioned in (a) above with respect to oral preparations. They can be prepared, for example, into powder, granules, pellets, tablets and various liquid preparations by mixing with or dissolving together with other food additives as required.

To the foods and drinks or food additives of the present invention may be added food additives generally employed in foods and drinks such as sweeteners, coloring agents, preservatives, thickening stabilizers, antioxidants; color developing agents, bleaching agents, fungicides, gum bases, bittering agents, enzymes, glazing agents, acidulants, seasonings, emulsifiers, nutrient supplements, additional materials for preparation, flavors and spice extracts.

To the foods and drinks of the present invention, an N-acylated hydroxyproline derivative or a salt thereof, or other active ingredients are added so as to be taken generally in an amount of 1-5000 mg, preferably 10-1000 mg, especially preferably 100-500 mg per adult person per day although the amount may vary depending upon the form of the foods and drinks.

These foods and drinks may be taken once or in several divided parts a day.

By taking them on daily basis, arthritis can be prevented.

The animal feeds of the present invention comprise an animal feed to which an N-acylated hydroxyproline derivative or a salt thereof is added. Those animal feeds that further comprise other active ingredients described above in connection with the foods and drinks are also included in the animal feeds of the present invention. The other active ingredients described in (a) above may also be added to them.

The animal feeds of the present invention include any feeds that prevent arthritis in non-human animals, preferably vertebrates, such as mammals other than humans, birds, reptiles, amphibians and fish, examples thereof being feeds for pets such as dogs, cats, rabbits and mice, pet food, feeds for livestock such as cows and pigs, feeds for poultry such as hens and turkeys, feeds for reptiles such as lizards, crocodiles and iguanas, feeds for amphibians such as frogs and newts and feeds for cultivated fish such as sea breams and young yellowtails.

The animal feeds of the present invention can be used as health supplement feeds for animals having an effect on preventing arthritis.

Except the addition of an N-acylated hydroxyproline derivative or a salt thereof, other active ingredients or a feed additive of the present invention to a feed, the animal feeds of the present invention can be prepared by using a conventional process for producing a feed.

The feeds include grains, bran, vegetable oil cakes, animal-based feed, other feeds and purified products, and mixtures thereof.

Examples of the grains are milo, wheat, barley, oats, rye, brown rice, buckwheat, foxtail millet, broomcorn millet, Japanese millet, corn and soybean.

Examples of the bran are rice bran, defatted rice bran, wheat bran, wheat middlings, wheat, germ, barley bran, pellet, corn bran and corn germ.

Examples of the vegetable oil cakes are soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake and mustard seed oil cake.

Examples of the animal-based feed are fish meal such as northern ocean meal, imported meal, whole meal and coastal meal, fish soluble, meat meal, meat and bone meal, blood powder, degradated hair, bone meal, treated by-products for livestock, feather meal, silkworm pupa, skim milk, casein and dry whey.

Examples of other feeds are stalks and leaves of plants such as alfalfa, hay cube, alfalfa leaf meal and powder of false acacia, by-products from the corn processing industry such as corn gluten, meal, corn gluten feed and corn steep liquor, processed starch products such as starch, products from the fermentation industry such as yeast, beer cake, malt root, alcohol cake and soy sauce cake, agricultural by-products such as processed citrus fruit cake, tofu cake, coffee cake and cocoa cake, cassava, broad bean, guar meal, seaweeds, krill, spirulina, chlorella and minerals.

Examples of the purified products are proteins such as casein and albumin, amino acids, sugars such as starch, cellulose, sucrose and glucose, minerals and vitamins.

The animal feeds of the present invention can also be produced by using granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method and extrusion methods using an extruding granulator or an extruder.

The feed additives of the present invention can be prepared according to methods similar to those mentioned in (a) above with respect to oral preparations. They can be prepared, for example, into powder, granules, pellets, tables and various liquid preparations by mixing with or dissolving together with other feed additives as required.

To the animal feeds of the present invention, an N-acylated hydroxyproline derivative or a salt thereof, or other active ingredients are added so as to be taken generally in an amount of 0.02-100 mg/kg, preferably 0.2-20 mg/kg, especially preferably 2-10 mg/kg although the amount varies depending upon the form of intake, the kinds of non-human animals to take them and the age and the body weight of the non-human animals.

The animal feeds are fed to non-human animals once or in several parts a day. It is also possible to administer the feed additives of the present invention as an oral preparation of a preventive against arthritis for non-human animals in the same dose and for the same period as in the case of the feeds described above.

A test example of the present invention examining the preventive effect of an N-acylated hydroxyproline derivative on arthritis is shown below.

### Test Example 1

### Effect of N-acetylhydroxyproline in mice with type II collagen-induced arthritis

It is known that arthritis is induced in DBA/1J mice (Charles River) by administering type II collagen two times.

As the first administration of type II collagen, a solution prepared, by mixing an equal amount of type II collagen [Collagen Gijutsu Kenshu-Kai (MCK)] and Freund's complete adjuvant (Iatron) and emulsifying the mixture using a homogenizer was intradermally administered to 6-week old male DBA/1J mice (Charles River) in an amount of 100 µl per one animal.

At day 21 after the first administration of type II collagen, as the second administration of type II collagen, a solution prepared by mixing an equal amount of type II collagen and Freund's complete adjuvant and emulsifying the mixture using a homogenizer in a similar manner was intradermally administered to the animals in an amount of 100 µl per one animal. In this manner, arthritis was induced in mice.

Starting on the day of the first administration of type II collagen, the mice were bred by feeding a powder feed, CE-2 (CLEA Japan), containing the addition of 0.02, 0.1 or 0.5% (hereinafter % refers to wt%) of N-acetylhydroxyproline (Kyowa Hakko Kogyo). As control, a group of mice were fed with the powder feed CE-2 containing no additive. At days 23, 27, 30, 33, 36, 40 and 42 after the first administration of type II collagen, the extent of the onset of arthritis was scored according to the following indices.

Scoring was carried out by applying 0-4 points with respect to one of four paws of mice: 0: no symptom; 1: swelling of one finger or swelling (slight) of ankles; 2: swelling of 1-3 fingers or swelling of ankles; 3: swelling of 3-5 fingers plus swelling of ankles; and 4: swelling of all the fingers plus swelling of ankles. Each mouse was scored the total of the points of 4 paws, namely, 0-16 points. Twenty mice each were subjected to observation with respect to each of the conditions.

The results are shown in Table 1. In the table, the figures show the score under different treatment conditions, which is given as mean ± SE (N=20).

In the table, "no treatment" means no treatment with type II collagen.

**Table 1**

| Score of arthritis under various treatment conditions | | | | | |
|---|---|---|---|---|---|
| | | N-Acetylhydroxyproline concentration (%) | | | |
| Days | No treatment | 0 | 0.02 | 0.1 | 0.5 |
| 0 | 0.0±0.0 | 0.0±0.0 | 0.0±0.0 | 0.0±0.0 | 0.0±0.0 |
| 23 | 0.0±0.0 | 0.1±0.1 | 0.0±0.0 | 0.1±0.1 | 0.0±0.0 |
| 27 | 0.0±0.0 | 6.0±0.7 | 4.2±0.8 | 4.6±0.9. | 3.8±0.7 |
| 30 | 0.0±0.0 | 8.5±0.8 | 7.6±0.9 | 7.3±1.0 | 5.9±0.9 |
| 33 | 0.0±0.0 | 9.9±0.7 | 9.3±0.8 | 8.3±1.1 | 7.7±0.8 |
| 36 | 0.0±0.0 | 10.4±0.7 | 9.7±0.9 | 8.6±1.0 | 8.1±0.8 |
| 40 | 0.0±0.0 | 10.3±0.7 | 10.0±0.8 | 8.5±1.0 | 8.4±0.7 |
| 42 | 0.0±0.0 | 10.5±0.7 | 10.1±0.9 | 8.7±1.0 | 8.5±0.7 |

As shown in Table 1, the scores of the mice who took the powder feed CE-2 containing the addition of 0.5%N-acetylhydroxyproline were significantly low compared with those of the mice who took the powder feed containing no addition of N-acetylhydroxyproline (control), showing that the addition of N-acetylhydroxyproline can suppress raising of the score of arthritis.

Likewise, a suppressive tendency of rising of the score of arthritis was observed for the mice who took the powder feed CE-2 containing the addition of 0.02% and 0.1% of N-acetylhydroxyproline, respectively, compared with the mice who took the powder feed CE-2 containing no additive.

From the foregoing, it was demonstrated that onset of arthritis could be suppressed by previously taking N-acetylhydroxyproline before the onset of arthritis.

Examples of the present invention are shown below.

### Best Modes for Carrying Out the Invention

In the following examples, N-acetylhydroxyproline, glucosamine and chondroitin sulfate produced by Kyowa Hakko Kogyo, Miyako Kagaku and Maruha Kagaku, respectively, were used.

### Example 1

Tablets of 8 mm in diameter and 200 mg in weight each were prepared by mixing the ingredients according to the composition shown in Table 2 below and tableting the resulting mixture using a tableting machine (Hata Seisakusho, HT-AP15SS-U).

**Table 2**

| Composition | Mixing rate (wt%) |
|---|---|
| N-Acetylhydroxyproline | 20 |
| Lactose | 40 |
| Calcium lactate | 10 |
| Magnesium stearate | 25 |
| Calcium carbonate | 5 |

### Example 2

Tablets of 8 mm in diameter and 200 mg in weight each were prepared by mixing the ingredients according to the composition shown in Table 3 below and tableting the resulting mixture using a tableting machine (Hata Seisakusho, HT-AP15SS-U).

**Table 3**

| Composition | Mixing rate (wt%) |
|---|---|
| N-Acetylhydroxyproline | 20 |
| Glucosamine | 20 |
| Lactose | 20 |
| Calcium lactate | 10 |
| Magnesium stearate | 25 |
| Calcium carbonate | 5 |

### Example 3

Tablets of 8 mm in diameter and 200 mg in weight each were prepared by mixing the ingredients according to the composition shown in Table 4 below and tableting the resulting mixture using a tableting machine (Hata Seisakusho, HT-AP15SS-U).

**Table 4**

| Composition | Mixing rate (wt%) |
|---|---|
| N-Acetylhydroxyproline | 15 |
| Glucosamine | 15 |
| Chondroitin sulfate | 15 |
| Lactose | 15 |
| Calcium lactate | 10 |
| Magnesium stearate | 25 |
| Calcium carbonate | 5 |

### Example 4

Tablets of 8 mm in diameter and 200 mg in weight each were prepared by mixing the ingredients according to the composition shown in Table 5 below and tableting the resulting mixture using a tableting machine (Hata Seisakusho, HT-AP15SS-U).

**Table 5**

| Composition | Mixing rate (wt%) |
|---|---|
| N-Acetylhydroxyproline | 20 |
| Chondroitin sulfate | 20 |
| Lactose | 20 |
| Calcium lactate | 10 |
| Magnesium stearate | 25 |
| Calcium carbonate | 5 |

### Example 5

A dog food was prepared according to the composition shown in Table 6.

**Table 6**

| Composition | Mixing rate (wt%) |
|---|---|
| N-Acetylhydroxyproline | 0.5 |
| Meat meal | 35.0 |
| Corn starch | 40.0 |
| Chicken extract | 5.0 |
| Yeast extract | 5.0 |
| Vegetable oil and fat | 5.0 |
| Calcium lactate | 1.0 |
| Sodium chloride | 1.0 |
| Sodium hydrogenphosphate | 0.5 |
| Magnesium carbonate | 0.5 |
| Ferrous sulfate | 0.1 |
| Vitamin B₁ | 0.0005 |
| Vitamin B₂ | 0.0005 |
| Vitamin E | 0.001 |
| Niacin | 0.005 |
| vitamin A | 2000IU |
| Vitamin D | 150IU |
| Moisture | 6.3 |

### Example 6

A drink is prepared according to the composition shown in Table 7 below.

**Table 7**

| Composition | Content (g) |
|---|---|
| N-Acetylhydroxyplorine | 49 |
| Pinedex #3 (Matsutani Chemical Industry) | 49 |
| Ferric pyrophosphate (iron source) | 0.1 |
| Phoscal EFC | 1.0 |
| (Calcium source: Nikko Fine Products) | |
| Vitamin mixture (Merck) | 1.0 |

The above mixture (20 g) is dispersed in 180 ml of water to prepare a drink.

### Example 7

A soft drink (10 bottles) is prepared from the ingredients shown in Table 8 below. Water is added to make the volume of 1000 ml.

**Table 8**

| Composition | Content (g) |
|---|---|
| N-Acetylhydroxyproline | 30 |
| Vitamin C | 1 |
| vitamin B₁ | 0.005 |
| Vitamin B₂ | 0.01 |
| Vitamin B₆ | 0.025 |
| Liquid sugar | 150 |
| Citric acid | 3 |
| Flavor | 1 |

### Example 8

A tea drink (1000 ml) is prepared by extracting the ingredients shown in Table 9 below with 1000 ml of water.

**Table 9**

| Composition | Content (g) |
|---|---|
| N-Acetylhydroxyproline | 30 |
| Tea leaves | 15 |

### Example 9

Cookies (30 pieces), are prepared from the ingredients shown in Table 10 below according to a conventional method.

**Table 10**

| Composition | Content (g) |
|---|---|
| N-Acetylhydroxyproline | 10 |
| Glucosamine | 10 |
| Chondroitin sulfate | 10 |
| Soft flour | 100 |
| Starch | 74 |
| Water | 14 |
| Baking powder | 2 teaspoonfuls |
| Salt | 1/2 teaspoonful |
| Egg | one |
| Butter | 80 |
| Milk | 2 tablespoonfuls |
| Honey | small amount |

### Example 10

A loaf of bread (4 pounds) is prepared from the ingredients shown in Table 11 below according to a conventional method.

**Table 11**

| Composition | Content (g) |
|---|---|
| N-Acetylhydroxyproline | 15 |
| Glucosamine | 15 |
| Strong flour | 1000 |
| Sugar | 50 |
| Salt | 20 |
| Skim milk | 20 |
| Shortening | 60 |
| Yeast (fresh) | 30 |
| Yeast food | 1 |
| Water | 650 |

### Example 11

Chewing gum (30 pieces) is prepared from the ingredients shown in Table 12 below according to a conventional method.

**Table 12**

| Composition | Content (g) |
|---|---|
| N-Acetylhydroxyproline | 1 |
| Gum base | 25 |
| Sugar | 63 |
| Starch syrup | 10 |
| Flavor | 1 |

### Example 12

Candies (20 pieces) are prepared from the ingredients shown in Table 13 below according to a conventional method.

**Table 13**

| Composition | Content (g) |
|---|---|
| N-Acetylhydroxyproline | 1 |
| Sugar | 80 |
| Starch syrup | 20 |
| Flavor | 0.1 |

### Example 13

Marmalade is prepared from the ingredients shown in Table 14 below according to a conventional method.

**Table 14**

| Composition | Content (g) |
|---|---|
| N-Acetylhydroxyproline | 5 |
| Chondroitin sulfate | 5 |
| Summer orange peel | 500 |
| Sugar | 200 |
| Summer orange juice | squeezed from |
| | one orange |

### Example 14

Hard capsules (360 mg/capsule) are prepared from the ingredients shown in Table 15 below according to the following method.

**Table 15**

| Composition | Content (mg) |
|---|---|
| N-Acetylhydroxyproline | 250 |
| Lactose | 60 |
| Corn starch | 30 |
| Hydroxypropyl cellulose | 20 |

N-Acetylhydroxyproline (250 mg) is mixed with 60 mg of lactose and 30 mg of corn starch, to which an aqueous solution of 20 mg of hydroxypropyl cellulose is added. The mixture is kneaded and then granulated according to a conventional method using an extruding granulator. The resulting granules are packed in gelatin hard capsules.

### Example 15

Soft capsules (170 mg/capsule) are prepared from the ingredients shown in Table 16 below according to the following method.

**Table 16**

| Composition | Content (mg) |
|---|---|
| N-Acetylhydroxyproline | 25 |
| Glucosamine | 25 |
| Soybean oil | 120 |

N-Acetylhydroxyproline (25 mg) and 25 mg of glucosamine are mixed with 120 mg of soybean oil. The mixture is packed in soft capsules according to a conventional method using a rotary dies automatic molding machine.

### Industrial Applicability

According to the present invention, it is possible to provide a preventive against arthritis, foods and drinks, food additives, animal feeds and feed additives comprising an N-acylated hydroxyproline derivative or a salt thereof as an active ingredient and a method for preventing arthritis in humans or non-human animals using them.

## Claims

1. A preventive against arthritis comprising an N-acylated hydroxyproline derivative or a salt thereof as an active ingredient.

2. The preventive against arthritis according to claim 1, wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

3. The preventive against arthritis according to claim 1 or 2, wherein the acyl moiety of said N-acylated hydroxyproline derivative is an acyl group having 2-23 carbon atoms.

4. The preventive against arthritis according to any one of claims 1 to 3, wherein said N-acylated hydroxyproline derivative is N-acetylhydroxyproline.

5. The preventive against arthritis according to any one of claims 1 to 4, wherein said arthritis is rheumatoid arthritis.

6. A food and drink or an animal feed comprising an N-acylated hydroxyproline derivative or a salt thereof.

7. The food and drink or the animal feed according to claim 6, wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

8. The food and drink or the animal feed according to claim 6 or 7, wherein the acyl moiety of said N-acylated hydroxyproline derivative is an acyl group having 2-23 carbon atoms.

9. The food and drink or the animal feed according to any one of claims 6 to 8, wherein said N-acylated hydroxyproline derivative is N-acetylhydroxyproline.

10. A food additive or a feed additive comprising an. N-acylated hydroxyproline derivative or a salt thereof.

11. The food additive or the feed additive according to claim 10, wherein said hydroxyproline is selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

12. The food additive or the feed additive according to claim 10 or 11, wherein the acyl moiety of said N-acylated hydroxyproline derivative is an acyl group having 2-23 carbon atoms.

13. The food additive or the feed additive according to any one of claims 10 to 12, wherein said N-acylated hydroxyproline derivative is N-acetylhydroxyproline.

14. Use of an N-acylated hydroxyproline derivative or a salt thereof for the production of a preventive against arthritis.

15. A method for preventing arthritis, which comprises administering an N-acylated hydroxyproline derivative or a salt thereof.
